(19) Europäisches Patentamt
European Patent Office
Office européen des brevets

(11) Veröffentlichungsnummer : **0 403 575 B1**

(12) # EUROPÄISCHE PATENTSCHRIFT

(45) Veröffentlichungstag der Patentschrift : **14.10.92 Patentblatt 92/42**

(51) Int. Cl.$^5$ : **A61K 31/205,** A61K 31/215, A61K 31/22, A01N 37/44

(21) Anmeldenummer : **89905609.7**

(22) Anmeldetag : **25.05.89**

(86) Internationale Anmeldenummer : **PCT/CH89/00097**

(87) Internationale Veröffentlichungsnummer : **WO 89/11276 30.11.89 Gazette 89/28**

(54) **ACYL-CARNITIN ZUR BEHANDLUNG UND ZUR VERHÜTUNG VON VIRUSINFEKTIONEN.**

(30) Priorität : **26.05.88 CH 1984/88**

(43) Veröffentlichungstag der Anmeldung : **27.12.90 Patentblatt 90/52**

(45) Bekanntmachung des Hinweises auf die Patenterteilung : **14.10.92 Patentblatt 92/42**

(84) Benannte Vertragsstaaten : **AT BE CH DE FR GB IT LI LU NL SE**

(56) Entgegenhaltungen :
**US-A- 4 415 588**
**W. Forth et al.: "Allgemeine und spezielle Pharmakologie und Toxikologie", 3. Auflage, 1980, Bibliographisches Institut,(Mannheim, DE), Seiten 526-528, Kapitel: "Antivirale Substanzen" siehe das ganze Kapitel, insbesondere Seite 528, rechte Spalte, 2.Absatz**
**Biochemical Pharmacology, Band 32, Nr. 22, 1983, Pergamon Press Ltd, (GB) R.J. Gilbert et al.: "Bromoacetyl-L-carnitine:biochemical and antitrypanosomal actions against Trypanosoma brucei brucei", Seiten 3447-3451**
**Cancer Research, Band 46, April 1986, T. Nakadate et al.: "Inhibition of 12-0-tetradecanoylphorbol-13-acetate-induced tumorpromotion and epidermal ornithine decarboxylase activity in mouse skin by palmitoylcarnitine", Seite 1589-1593 sieheZusammenfassung**
**Annals of Internal Medicine, Band 87, 1977, B.M. Patten et al.: "Hepatitis-associated lipid storage myopathy", Seiten 417-421siehe Zusammenfassung; Seite 419, rechte Spalte, vorletzter Absatz**

(56) Entgegenhaltungen :
**J. Drug Dev., Band 1, Nr. 3, November 1988, C. De Simone et al.: "Amelioration of the depression of HIV-infected subjectswith L-acetyl carnitine therapy", Seiten 163-166 siehe Zusammenfassung**

(73) Patentinhaber : **Bernardini, Attilio**
**Tösstalstrasse 254**
**CH-8405 Winterthur (CH)**

(72) Erfinder : **SCANDURRA, Laura**
**Via Duca d'Aosta, 1**
**I-95029 Viagrande (IT)**
Erfinder : **AURELIAN, Laure**
**3404 Bancroft Road**
**Baltimore, MD 21215 (US)**

(74) Vertreter : **Justitz-Wormser, Daisy P., Dipl.-Chem. et al**
**Patentanwalts-Bureau ISLER AG Postfach 6940**
**CH-8023 Zürich (CH)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 403 575 B1

Jouve, 18, rue Saint-Denis, 75001 PARIS

**Beschreibung**

Viren verschiedenster Art sind eine grosse gesundheitliche Gefährung für Menschen, Tiere und Pflanzen und können zu harmlosen bis höchst gefährlichen Infektionen führen. Im Gegensatz zu Bakterien benötigen Viren zum Leben, zur Entfaltung ihrer Aktivität und zur Vermehrung eine lebende Wirtszelle.

In neuerer Zeit trat, mit den Aenderungen der Sexualgebräuche, eine beunruhigende Zunahme von sexuell übermittelten Virusinfektionen, wie mit Herpes simplex Virus (HSV) -Typus 1 und 2, Cytomegalo-Virus (CMV), humanem Immunodefizienz-Virus (HIV), humanem Papilloma-Virus (HPV), usw. auf.

Gegenwärtig stehen keine weitreichend befriedigenden antiviralen Arzneimittel zur Verfügung, mit Ausanhme von Acyloguanosin ("Acylovir"), welches bei der Behandlung von Herpes verwendet wird, und Aziotimidin (AZT), welches bei der Behandlung von Aids eingesetzt wird.

Keines dieser Arzneimittel ist problemlos. Hauptsächlich aber sind präventive Modalitäten nicht erhältlich.

Die Prävention wäre insbesondere wünschenswert in Fällen, wie HSV, CMV, HIV und VCV, bei welchen der Virus während der Lebenszeit des Wirtes fortbsteht und oft eine Erkrankung viele Jahre später und von verschiedener Pathologie hervorruft.

Alle bisher vorgeschlagenen Mittel bezweckten eine direkte Bekämpfung des Virus nach Eintritt in die Zelle, um ihn dort zu inaktivieren oder abzutöten.

Aufgabe der Erfindung war es daher, ein Mittel zu finden, welches Viren wirksam bekämpft ohne eine schädliche Nebenwirkung für Menschen, Tiere und Pflanzen auszuüben.

Ueberraschenderweise wurde nun gefunden, dass L-Acyl-carnitin zur Herstellung eine Arzneimittels verwendet, werden kann, welches nicht nur auf die Virusreplikation direkt einwirkt, sondern den Virus durch eine noch unaufgeklärte Veränderung der Wirtszelle vollständig inaktiviert und ihm das Fortleben verunmöglicht.

Die Erfindung betrifft die Verwendung von L-Acyl-carnitin zur Herstellung eines Arzneimittels zur Behandlung und zur Verhütung von Virusinjecktionen bei Menschen, Tieren und Pflanzen. Besondere Ausführungsformen der Erfindung sind aus den abhängigen Ansprüchen zu entnehmen.

Die bevorzugte Verbindung, welche als wirksam in dieser Beziehung gefunden wurde, ist L-Acetylcarnitin (γ-Trimethyl-β -acetyl-butyrobetain) der Formel :

$$(CH_3)_3-N^+-CH_2-\underset{\underset{OCOCH_3}{|}}{CH_2}-CO_2-COOHCl^+$$

Carnitin ist eine natürlich im Körper vorkommende Verbindung. Auch das ortho-L-Acetylderivat ist ein bekanntes, sowohl in verschiedenen Organen vorkommendes, wie auch synthetisches, im Handel erhältliches Derivat, das z.B. für orale Verwendung in Form von Pillen oder Tropfen zur Behandlung altersbedingter neurologischer, metabolischer Störungen empfohlen wird. Solche Störungen sind z.B. Gedächnisverlust, befremdliches Verhalten und erratische Erscheinungen, wie sie üblicherweise bekannt sind als Demantia senilis.

Ferner waren Bromacetyl-carnitin und Palmitoylcarnitin als Antitryp anosom- bzw. Antitumor-Wirksubstanzen bekannt.

Es wurde nun völlig unerwarteterweise festgestellt, dass insbesondere L-Acetyl-carnitin eine potente antivirale Aktivität ausübt und einerseits zur Behandlung viraler Infektionen, insbesondere aber auch zur Verhütung solcher Infektionen mit erstaunlichem Erfolg eingesetzt werden kann.

Die Untersuchungen, welche durchgeführt wurden, um die antivirale Aktivität von L-Acetyl-carnitin nachzuweisen, können wie folgt zusammengefasst werden:

1] Wirkungen von L-Acetyl-carnitin auf das Viruswachstum in Gewebekulturen

Zwei Versuchsserien wurden durchgeführt. In der ersten Serie wurden verschiedene Zell-Linien von tierischem und menschlichem Ursprung (afrikanischer Grünaffe - African green monkey; menschliche cervikale Krebszellen) mit verschiedenen Viren, einschliesslich HSV Typus 1 und 2, CMV, Adeno-Virus, Varicella-Zoster-Virus (VZV), Respiratory syncytial Virus (RSV), Polio-Virus, Coxsackie-Virus, Entero-Virus und Vaccinia-Virus infisziert.

Je fünf Kulturen, welche mit den obigen Viren infisziert waren, wurden mit je 100 mg/ml L-Acetyl-carnitin.HCl oder Kontrollmedium (kein Wirkstoff) behandelt.

Die Virustiter wurden in täglichen Intervallen während 12 Tagen bestimmt.

Alle Kontrollen reagierten positiv auf das Viruswachstum mit Titern im Bereich zwischen $10^4$ bis $10^8$ kolo-

niebildenden Einheiten (plaque forming units pfu) /ml je nach dem spezifischen Virus.

Alle mit L-Acetyl-carnitin.HCl behandelten Proben waren hingegen negativ (0 pfu/ml), d.h. es trat keine Vermehrung der Viren ein.

In der zweiten Versuchsreihe wurde die Wirkung der L-Acetyl-carnitin-Dosierung auf die antivirale Aktivität bestimmt.

Je fünf Kulturen wurden infisziert und behandelt mit 10, 20, 30, 40, 50, 60, 70, 80, 90, 100, 200, 400 mg/ml an L-Acetyl-carnitin.HCl oder an Kontrollmedium und die Virustiter wurden wie oben bestimmt.

Die antivirale Aktivität wurde bei allen Dosierungen von mehr als 10 bis 20 mg/ml festgestellt. Die antivirale Aktivität trat entweder vollständig oder gar nicht auf und zeigte kein dosisabhängiges Muster.

2] Wirkungen der Einwirkungszeit auf die antivirale Aktivität

Diese Versuche bezweckten, die Zeit zu bestimmen, während welcher eine Infektion mit HSV1 oder HSV2 durch L-Acetyl-carnitin.HCl verhindert wird.

Je fünf Kulturen von infiszierten Zellen wurden behandelt (40 mg/ml) zu den folgenden Zeiten :
-24 Stunden, -12 Stunden, -2 Stunden, -1 Stunde, -30 Minuten, -20 Minuten, -10 Minuten, 0, 1 Stunde, 2 Stunden, 3 Stunden, 4 Stunden, 8 Stunden, 12 Stunden und 16 Stunden nach der Infektion.

Sie wurden jeweils 24 Stunden nach der Infektion auf das Viruswachstum untersucht.

Die Virusreplikation war in allen Kulturen, welche zwischen 24 Stunden vor bis 4 Stunden nach der Infektion behandelt worden waren, vollständig unterbunden.

Die Viren vermehrten sich normal in Kulturen, welche später als 4 Stunden nach der Infektion mit L-Acetyl-carnitin behandelt worden waren.

Bei diesen Untersuchungen war die Verhinderung des Viruswachstums absolut (0 pfu/ml), während sich die Viren in Kontrollen, welche nicht mit L-Acetyl-carnitin behandelt worden waren oder diese Behandlung später als 4 Stunden nach der Infektion erhielten, normal vermehrten.

3] Minimale Einwirkungszeit, welche für die antivirale Aktivität erforderlich ist

Je fünf Kulturen wurden der Einwirkung von L-Acetyl-carnitin.HCl (40 mg/ml) während 10 Minuten bzw. 20 Minuten, 30 Minuten und 60 Minuten ausgesetzt. Das L-Acetyl-carnitin.HCl wurde sodann entfernt und die Zellen mit HSV1 oder HSV2 entweder in diesem Zeitpunkt oder 24 bzw. 48 oder 72 Stunden später infiszieit.

Das Viruswachstum wurde vollständig unterbunden, wenn die Einwirkung von L-Acetyl-carnitin-HCl während mindestens 20 Minuten erfolgte, selbst wenn die Infektion erst 3 Tage später erfolgte.

4] Wirkung von L-Acetyl-carnitin auf die Virusabsorption

Die Beobachtung, dass L-Acetyl-carnitin 4 Stunden nach der Infektion mit HSV noch voll wirksam ist, legt es nahe, dass es auch wirksam ist nach der ersten Interaktion des Virus mit der Zelle.

Da bereits die Vorbehandlung der Zellen mit L-Acetyl-carnitin das Viruswachstum so erfolgreich inhibiert, wurde untersucht, ob L-Acetyl-carnitin auch die Adsorption inhibiert.

Zellen wurden mit L-Acetyl-carnitin (40 mg/ml) 1 Stunde vor oder gleichzeitig mit der Injektion behandelt.

Die Infektion erfolgte mit Viren, welche mittels tritiiertem Timidin radioaktiv markiert worden waren, und das Auftreten der Markierung wurde im Cytoplasma und der Membranfraktion der Zellen als eine Funktion der Abnahme aus dem infektiösen Ueberstehenden verfolgt.

Die Markierung wurde in der Membran und in der Cytoplasmafraktion der infiszierten Zellen in einem kinetischen Muster, welches mit bekannten Daten übereinstimmte, wiedergefunden, was auf die erfolgte Virus-Adsorption und -Penetration hinweist.

5] Stabilität der Wirksamkeit von L-Acetyl-carnitin gegen Temperaturveränderungen

L-Acetyl-carnitin.HCl (40 mg/ml) wurde den folgenden Behandlungen unterzogen:
(1) Keine Behandlung
(2) 1 Woche Stehenlassen bei Zimmertemperatur
(3) 30 Minuten bei 56°C
(4) 2 Stunden bei 37°C
(5) sieben Gefrier-Auftau-Zyklen.

Nach diesen Vorbehandlungen wurde jede der Lösungen auf Zellkulturen, welche mit HSV infisziert worden waren, zur Einwirkung gebracht und zwar während 1 Stunde bei 37°C. 24 Stunden nach Beginn dieser

Einwirkung wurde der Virustiter in jeder Probe bestimmt. Alle fünf Präparate inhibierten das Viruswachstum vollständig (0 pfu/ml) während entsprechende Kontrollen (ohne L-Acetyl-carnitin) ein ungehindertes Wachstum der Viren ($10^7$ pfu/ml) ergaben.

Daraus kann geschlossen werden, dass die antivirale Aktivität von L-Acetyl-carnitin unter den oben angeführten Temperaturbedingungen völlig stabil ist.

6] Direkte Wirkung von L-Acetyl-carnitin auf Viren

Um festzustellen, wie gross die Wirksamkeit von L-Acetyl-carnitin auf den Virus selbst ist, d.h. ob der Virus selbst von der Substanz angegriffen wird und nicht infolge Veränderung der Wirtszelle am Fortleben gehindert wird, wurden tritiierte HSV-Viren in einem Reagensglas ohne Zellen der Einwirkung von L-Acetyl-carnitin.HCl (40 mg/ml) bzw. einer Kontrolle ohne Wirkstoff während 1 Stunde bei 37°C unterworfen. Die Viren wurden sodann über ein Milliporfilter (0,22 µ Porendurchmesser) vakuumfiltriert. Die auf dem Filter verbleibende Radioaktivität wurde gemessen, da nur die intakten Viren auf dem Filter zurückgehalten werden, während angegriffene, solubilisierte Viren, die in ihre molekularen Bestandteile zerfallen sind, durch den Filter abgezogen werden.

Nach Behandlung der Viren mit dem Kontrollpräparat wurde die volle ursprüngliche Radioaktivität auf dem Filter vorgefunden. Nach Behandlung mit L-Acetyl-carnitin betrug die gemessene Radioaktivität nur noch 50 % der ursprünglich gemessenen. Daraus lässt sich schliessen, dass die direkte, strukturschädigende Wirkung von L-Acetyl-carnitin auf den Virus selbst nicht unbedeutend ist.

7] Wirkung von L-Acetyl-carnitin auf herpetische Hautläsionen

Diese Untersuchungen dienen der Bestimmung der Wirkung der L-Acetyl-carnitin-Behandlung auf die Entwicklung von klassischen herpetischen Erkrankungen an der Maus.

Verdünnungen von L-Acetyl-carnitin.HCl in einem Polyäthylenglykol-Träger (PEG) wurden zubereitet. Dies ergab ein wirksames Gel für örtliche Anwendung. Die L-Acetyl-carnitin-Dosen im Gel betrugen 0,5 bzw. 1, 2 und 4 g/ml.

Mäuse wurden mit L-Acetyl-carnitin enthaltenden Gelen durch örtliche Anwendung auf das Ohr bei -2, 0 und +2 Stunden nach der Infektion mit HSV1 oder HSV2 durch subkutane Injektion in das behandelte Ohr behandelt.

Kontrollen bestanden aus mit PEG ohne L-Acetyl-carnitin behandelten und auf ähnliche Weise infiszierten Mäusen.

Eine zusätzliche Behandlung erfolgte 24 Stunden später durch ähnliche örtliche Anwendung des entsprechenden Gels.

Alle unbehandelten Mäuse sowie jene, welche mit 500 mg/ml behandelt worden waren, entwickelten 3 Tage später HSV-Läsionen (10/10 - 100 %), keine der mit der 4 g/ml -Salbe behandelten Mäuse entwickelte eine Erkrankung im Laufe von 8 Tagen nach der Infektion (0/10 - 0 %).

Ein Drittel (33 %) der mit der 1 g/ml- und ein Fünftel (20 %) der mit der 2 g/ml-L-Acetyl-carnitin-Salbe behandelten Tiere zeigten minimale Erkrankungssymptome 6 Tage nach der Infektion.

Die anderen blieben frei von Erkrankungen während der ganzen 8 Tage.

Die Ohren wurden entfernt und die Virustiter am Ende der Untersuchung (8 Tag) bestimmt.

Die Virustiter in den Ohren der Kontrollgruppe lagen im Bereich zwischen 1 und 4 x $10^4$ pfu/ml.

Die zwei Tiere, welche mit 1 bzw. 2 g/ml-L-Acetyl-carnitin-Salbe behandelt worden waren, und welche minimale Erkrankungserscheinungen aufwiesen, hatten Titer von 1 bzw. 5 x $10^3$ pfu/ml.

Die anderen sechs Tiere ohne Erkrankungssymptome, welche mit 1 bzw. 2 g/ml-L-acetyl-carnitin-Salben behandelt worden waren, wiesen Titer von 1 bis 3 x $10^2$ pfu/ml auf.

Es war kein Virus (0 pfu/ml) in Tieren, welche mit 4 g/ml Salbe behandelt worden waren

8] Toxizität

L-Acetyl-carnitin.HCl weist für Mäuse eine akute Toxizität $DL_{50}$ von über 3000 mg/kg bei intramuskulärer Verabreichung, von über 3600 mg/kg bei interperitonealer Verabreichung, von über 1600 mg/kg bei intravenöser Verabreichung und von über 18'000 mg/kg bei oraler Verabreichung auf. Für Ratten betragen diese Werte über 3000 mg/kg i.m. bzw. 2748 mg/kg i.p., 1000 mg/kg i.v. und über 10'000 mg/kg p.o.

Langzeitversuche mit Meerschweinchen und Kaninchen, denen während 26 Wochen L-Acetyl-carnitin in Dosen von 250 bis 500 mg/kg/Tag per os und in Dosen von 50 mg/kg/Tag i.m. verabreicht wurde, zeigten keine wesentlichen Veränderungen des Gewichtes, der Blutzusammensetzung, der Leberfunktion, der biochemischen Werte des Blutes und des Urins. Auch die macro-microskopischen Untersuchungen der wichtigsten Organe ergaben keine pathologische Veränderung.

Auch die Untersuchung an Hunden ergaben nach i.m.-Verabreichung von 75, 150 und 300 mg/kg/Tag keinerlei Anzeichen toxikologischer Natur.

Zusätzlich wurde beobachtet, dass die örtliche Anwendung Vasodilatation erzeugt. Topische Anwendung auf die Haut der Hand von 12 menschlichen Freiwilligen in einer Konzentration von 4 mg/ml ergab, dass die Verbindung nicht brennt, sticht oder schmerzt Es erzeugte jedoch einen minimalen, angenehmen Wärmeeffekt, voraussichtlich infolge Vasodilatation.

In Gewebezellkulturen ist L-Acetyl-carnitin minimal toxisch, insbesondere inhibiert es die Proteinsynthese der Zellen oberhalb einer Konzentration von 40 mg/ml.

In der örtlich behandelten Maus war jedoch keine sichtbare Toxizität mit Ausnahme der auftretenden lokalen etwas rötlichen Färbung, welche bei einer Dosis von 4 g/ml beobachtet wurde und voraussichtlich ebenfalls auf Vasodilatation zurückzuführen ist.

Zusammenfassend zeigen die Daten, dass sowohl in Gewebekulturen wie in Tieren L-Acetyl-carnitin das Wachstum aller untersuchten Viren verhindert. Spezifisch macht die Behandlung die Zelle offensichtlich refraktorisch gegen Infektion und scheint dadurch allen Viren die Lebens- und Vermehrungsfähigkeit zu rauben. Die Wirkung, welche sie auf die Infektionen mit HIV ausübt, konnte noch nicht direkt studiert werden, weil dieser Virus sehr schwer in Zelltypen zu züchten ist. Der Versuch muss daher direkt am Menschen durchgeführt werden.

Gestützt auf die Erfahrung mit allen anderen sehr verschiedenartigen Virus-Typen wird jedoch erwartet, dass das Mittel auch den Eintritt von HIV in die Körperzellen verhindern wird, sofern die Uebertragung von HIV durch direkten Kontakt, z.B. während des Sexualverkehrs, erfolgt. Vor allem wird vorgeschlagen, den Wirkstoff für topische Anwendungen in einem Crème-Träger zu verwenden, dessen Formulierung allgemein bekannt ist.

In den obigen Untersuchungen wurde der Wirkstoff in Lösung in einem, im Handel erhältlichen und den Fachleuten gut bekannten Zellwachstumsmedium, nämlich "Eagel's MEM" (MEM = Minimum Essential Medium), gegebenenfalls unter Zusatz von 1 % Kalbsfoetus-Serum, verwendet.

Das L-Acetyl-carnitin.HCl ist in Wasser und den in Kosmetika üblichen Lösungsmitteln löslich. Die wässerige Lösung weist mit steigender Konzentration eine zunehmende Viskosität auf. So ist bereits eine Lösung von 4 g L-Acetyl-carnitin in 1 ml Wasser sehr stark viskös.

Zwei Typen von Crèmen können bevorzugt in Betracht gezogen werden, die sich auf übliche Art herstellen lassen :

Beispiel 1

Crème für äussere Anwendung zur Behandlung von z.B. herpetischen Läsionen, welche nach Einwirkung von Sonne, UV-Licht, Fieber auftreten können.

Der Träger enthält : Aethylalkohol 15 %, Carboxypolymethylen 2 %, EDTA 0,1 %, Lavendelessenz 0,0075 %. Diesem Träger werden 10 mg bis 4 g/ml L-Acetyl-carnitin.HCl zugesetzt.

Der Geruch des Präparates kann mit jedem zur Verfügung stehenden Parfum, wie Vanille, Jasmin, Erdbeer, Moschus, usw. nach Wunsch geändert werden.

Diese Salbe ergibt bei Anwendung auf die erkrankte Stelle eine völlige Abheilung bereits nach 2 Tagen, während übliche Mittel cirka 10 Tage benötigen Die Salbe dient ebenso dem zuverlässigen Schutz aller damit bestrichenen Hautpartien vor Ansteckung.

Die Anwendung der Präparate in flüssiger oder halbflüssiger Form erfolgt ähnlich wie bei einem Sonnenschutzmittel. Die zu behandelnde Partie wird mit einer dünnen Schicht des Präparates vollständig und gleichmässig bedeckt.

Beispiel 2

Der zweite Salbentypus, welcher zur Verwendung vorgeschlagen wird, wird mit oder ohne Zusatz eines Spermicides in einem inerten Trägergel bekannter Art zur intravaginalen Verwendung als gegebenenfalls contraceptives antivirales Präparat hergestellt.

Am zweckmässigsten erfolgt die Anwendung solcher Präparate unmittelbar vor dem Geschlechtsverkehr und einmal danach.

Die Salben können auch auf anderen Körperpartien verwendet werden, wie z.B. dem Anus während analem Geschlechtsverkehr.

Beispiel 3 Vaginalcrème

| | |
|---|---|
| Sorbitolmonostearat | 2 % |
| Polysorbitol 60 | 1,5 % |
| Spermoid | 2 bis 3 % |
| Cetylstearylalkohol | 10 % |
| 2,8-Octyl-dodecanol | 13,5 % |
| Benzylalkohol | 1 % |
| L-Acetyl-carnitin.HCl | 1 bis 20 % |
| Rest | Wasser |

Anstelle von Salben kann der Wirkstoff auch in anderer vorzugsweise topisch wirksamer Form angewandet werden, z.B. als Lotion, Suppositorien, Spray, Vaginalkapseln oder -zäpfchen, usw.

Beispiel 4 Analcrème

| | |
|---|---|
| Methyl-p-hydrobenzoat | 7 % |
| Propyl-p-hydrobenzoat | 3 % |
| L-Acetyl-carnitin.HCl | 1 bis 20 % |
| Gemische von Polyäthylenglykol 400 und Polyäthylenglykol 4000 im Verhältnis 2 : 1 | Rest |

Beispiel 5

Vaginalzäpfchen

| | |
|---|---|
| Benzoesäure | 0,5 g |
| Glyeryl-monorhicinoleat | 0,2 g |
| halbsynthetische Glyceride | 2,35 g |
| L-Acetyl-carnitin.HCl | 10 mg bis 4 g |

Anwendungen in anderen pharmazeutischen Dosierungsformen, z.B. per os, intraperitoneal, intramuskulär oder intravenös werden gegenwärtig überprüft. Ebenso sind Versuchsreihen zur Bekämpfung von Virusinfektionen an Tieren und Pflanzen im Gange.

## Patentansprüche

1. Verwendung von L-Acyl-carnitin zur Herstellung eines Arzneimittels zur Behandlung und zur Verhütung von Virusinfektionen bei Menschen, Tieren und Pflanzen.

2. Verwendung nach Anspruch 1 von L-Acetyl-carnitin.

3. Verwendung nach Anspruch 1 oder 2 zur Herstellung eines topischen Präparates, z.B. einer Salbe, einer Lotion, eines Sprays, eines Vaginalzäpfchens, einer Vaginalkapsel oder eines Suppositoriums.

4. Verwendung nach Anspruch 3 zur Herstellung eines Präparates, welches den Wirkstoff in einer Konzentration von 10 mg bis 4 g/ml bzw. pro Gramm enthält.

5. Verfahren zur Behandlung oder zur Verhütung von Virusinfektionen bei Pflanzen, dadurch gekennzeichnet, dass man ein Mittel, erhalten nach einem der Ansprüche 1 oder 2, in wirksamer Dosierung zur Anwendung bringt.

## Claims

1. Use of L-acyl-carnitine for the production of a therapeutic agent for treating and preventing viral infections in humans, animals and plants.

2. Use according to claim 1 of L-acetyl-carnitine.

3. Use according to claim 1 or 2 for the production of a topic preparation, e.g. an ointment, a lotion, a spray, a viral suppository, a vaginal capsule or a suppository.

4. Use according to claim 3 for the production of a preparation containing the active substance in a concentration of 10 mg to 4 g per ml or per gramme respectively.

5. Method for trating or preventing viral infections in plants, characterized in that a preparation obtained according to claim 1 or 2 is applied in an efficacious dosage.

## Revendications

1. Utilisation de la L-acyle-carnitine pour la production d'un médicament pour le traîtement et la prévention d'infections virales chez les hommes, les animaux et les plantes.

2. Utilisation selon la revendication 1 de la L'acétyle-carnitine.

3. Utilisation selon la revendication 1 ou 2 pour la production d'une préparation topique, par exemple un onguent, une lotion, un spray, un suppositoire vaginal, une capsule vaginale ou un suppositoire.

4. Utilisation selon la revendication 3 pour la production d'une préparation contenant le princip activ dans une concentration de 10 mg à 4 g/ml ou par gramme respecivement.

5. Procédé pour le traîtement ou la prévention d'infections virales chez les plantes, caractérisé en ce qu'on applique une préparation obtenu selon l'une des revendications 1 ou 2 en dosage effectif.